(19) Europäisches Patentamt
European Patent Office
Office européen des brevets

(11) Numéro de publication: 0 223 701 B1

(12) # FASCICULE DE BREVET EUROPEEN

(45) Date de publication de fascicule du brevet: **25.03.92**

(51) Int. Cl.[5]: **A61K 31/48**

(21) Numéro de dépôt: **86402506.9**

(22) Date de dépôt: **12.11.86**

(54) **Utilisation de dérivés de l'ergoline à l'obtention d'un médicament à protecteur neuronal.**

(30) Priorité: **13.11.85 FR 8516743**

(43) Date de publication de la demande:
**27.05.87 Bulletin 87/22**

(45) Mention de la délivrance du brevet:
**25.03.92 Bulletin 92/13**

(84) Etats contractants désignés:
**CH DE FR GB IT LI NL**

(56) Documents cités:
**CH-A- 609 982**
**CH-A- 657 366**
**GB-A- 2 125 041**
**US-A- 4 246 265**

**MEDICINAL RESEARCH REVIEWS, vol. 3, no. 3, 1983, pages 237-257, John Wiley & Sons; J.H. GROWDON: "Neuropharmacology of degenerative diseases associated with aging"**

**JOURNAL DE PHARMACOLOGIE, vol. 16, suppl. III, 1985, pages 21-27, Paris, FR; M. GOLDSTEIN: "Alcaloides de l'ergot et récepteurs centraux monoaminergiques"**

(73) Titulaire: **ROUSSEL-UCLAF**
**35, Boulevard des Invalides**
**F-75007 Paris(FR)**

(72) Inventeur: **Nedelec, Lucien**
**45, boulevard de L'Ouest**
**F-93340 -Le Raincy(FR)**

(74) Mandataire: **Vieillefosse, Jean-Claude**
**Département des Brevets ROUSSEL UCLAF**
**B.P no 9**
**F-93230 Romainville(FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet l'utilisation de derivés de l'ergoline à l'obtention d'un médicament protecteur neuronal.

Un grand nombre de dérivés de synthèse de l'ergoline sont connus pour leurs propriétés dopaminergiques : antiprolactine, hypotensives ou antihypertensives ou antiparkinsonniennes.

Ces dérivés et leurs activités sont décrits notamment dans les brevets belges n° 858 633, 849 318, 802 531, 811 610, 794 888 pour le brevet US n° 3 901 894 ou 4 246 265.

Certains dérivés de l'ergoline sont également connus pour lutter contre la démence sénile, l'insuffisance cérébrale et sont utilisables en gériatrie (cf. GB 2125041 ou CH 609982).

La demanderesse, en poursuivant les recherches sur cette série, a découvert de manière tout à fait inattendue que certains de ces dérivés étaient doués de remarquables propriétés utilisables en gériatrie pour compenser le vieillissement ou la souffrance cérébrale. Ces propriétés, la connaissance de la demanderesse, n'ont aucun rapport ou lien de parenté avec les propriétés dopaminergiques.

C'est ainsi que la présente demande a pour objet l'utilisation des dérivés de l'ergoline de formule (I) :

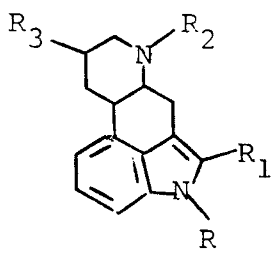

(I)

dans laquelle R représente un atome d'hydrogène ou un radical alkyle renfermant de 1 à 4 atomes de carbone, $R_1$ représente un atome d'hydrogène, de chlore ou de brome, $R_2$ représente un atome d'hydrogène ou un radical alkyle renfermant de 1 à 4 atomes de carbone, $R_3$ représente un radical alkyl ($C_1$-$C_4$) thiométhyle, ainsi que de leurs sels d'addition avec les acides minéraux ou organiques pharmaceutiquement acceptables à l'obtention d'un médicament protecteur neuronal.

Dans la formule générale I et dans ce qui suit, le terme radical alkyle renfermant de 1 à 4 atomes de carbone désigne de préférence un radical méthyle, éthyle, propyle ou isopropyle ; le terme alkyl ($C_1$-$C_4$) thiométhyle désigne par exemple le radical n-propyl-, éthyl- ou de préférence méthylthiométhyle.

Les sels d'addition avec les acides minéraux ou organiques peuvent être par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Pour leur utilisation comme protecteurs des neurones, on retient de préférence les dérivés de formule (I) dans laquelle R représente un atome d'hydrogène, ainsi que leurs sels d'addition avec les acides minéraux ou organiques pharmaceutiquement acceptables. Parmi ces derniers, on retient plus particulièrement ceux pour lesquels $R_1$ représente un atome d'hydrogène ainsi que leurs sels d'addition avec les acides minéraux ou organiques pharmaceutiquement acceptables, et tout particulièrement la 6-méthyl 8$\beta$-(méthylthiométhyl) ergoline ainsi que ses sels d'addition avec les acides minéraux ou organiques pharmaceutiquement acceptables. On peut citer également la 6-méthyl 8$\beta$-(méthylthiométhyl) 9,10-didéhydroergoline ainsi que ses sels d'addition avec les acides minéraux ou organiques pharmaceutiquement acceptables.

Les dérivés de l'ergoline de formule I ainsi que leurs sels sont doués de remarquables propriétés protectrices des neurones.

Ils peuvent ainsi être utilisés notamment dans le traitement de la sénescence cérébrale ou des manifestations liées à une hypoxie cérébrale.

La dose usuelle, variable selon le dérivé utilisé, le sujet et l'affection en cause peut être, par exemple, de 1 mg à 200 mg par jour. Par voie orale, chez l'homme, le dérivé de l'exemple peut être administré à la dose quotidienne de 1 mg à 100 mg, par exemple, pour le traitement de la sénescence cérébrale, soit environ de 0,015 mg à 1,5 mg par kilogramme de poids corporel.

L'invention a enfin pour objet les compositions pharmaceutiques protectrices des neurones qui renferment au moins un dérivé précité ou l'un de ses sels d'addition avec les acides pharmaceutiquement

acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émusifiants, les conservateurs.

L'exemple qui suit illustre la présente invention, sans toutefois la limiter.

Exemple:

Etude pharmacologique du chlorhydrate de la 6-méthyl 8$\beta$ -(méthylthiométhyl) ergoline (produit A) et du dérivé 9,10-didéhydroergoline correspondant (produit B) décrits dans le brevet US n¤ 3 901 894.

a - Test de l'anoxie hypobare :

On utilise des souris mâles d'un poids de 20-22 g, à jeun depuis cinq heures, réparties par groupes de 10 animaux.

On administre aux animaux le produit à tester, par voie souscutanée. Quinze minutes après administration du produit, on place les animaux dans un dessicateur de 2 litres dans lequel on amène rapidement la pression à 25.33 kPa (190 mm de Hg), au moyen d'une pompe, et on note leur temps de survie exprimé en secondes.

On calcule l'augmentation du temps de survie, exprimé en pourcentage, des animaux traités par rapport aux animaux témoins, soumis aux mêmes conditions.

On a obtenu les résultats suivants :

| Produit | Doses mg/kg (S.C.) | Augmentation du temps de survie |
|---|---|---|
| A | 1<br>10 | + 12%<br>+ 57% |

b - Test de l'énolase :

Les cellules cérébrales en souffrance libèrent de l'énolase $\gamma\gamma$ qui est un marqueur spécifique de lésion neuronale. Dans le test, les lésions sont réalisées chez la souris par injection sous cutanée de 35 mg/Kg d'acide kaïnique. Le produit A administré par voie intrapéritonéale à la dose de 1 mg/Kg, 1 heure avant l'injection du neurotoxique, diminue de 29 % la concentration sérique d'énolase. Par conséquent, ce produit protège les cellules cérébrales en situation de souffrance.

c - Etude de la toxicité aiguë.

On a évalué la dose létale $DL_0$ du produit A après administration par voie orale chez la souris.

On appelle $DL_0$ la dose maximale ne provoquant aucune mortalité en 8 jours.

Le résultat obtenu est le suivant : $DL_0$ en mg/kg = 200.

d - Test de l'anoxie asphysique.

Les souris sont curarisées et placées sous assistance respiratoire. On administre le produit à tester à la dose de 2 mg/kg, et supprime l'assistance respiratoire. On note l'augmentation du temps nécessaire à la disparition de l'électroencéphalogramme, par rapport aux animaux témoins.

Les résultats sont les suivants :

| Produit | administration observée |
|---|---|
| A<br>B | + 10 %<br>+ 11 % |

Les produits A et B prolongent la survie électro-encéphalographique chez les animaux traités.

**Revendications**

1. L'utilisation des dérivés de l'ergoline de formule (I) :

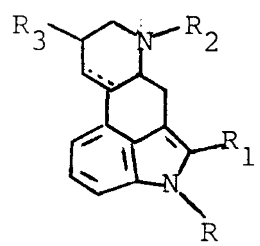

(I)

dans laquelle R représente un atome d'hydrogène ou un radical alkyle renfermant de 1 à 4 atomes de carbone, $R_1$ représente un atome d'hydrogène, de chlore ou de brome, $R_2$ représente un atome d'hydrogène ou un radical alkyle renfermant de 1 à 4 atomes de carbone, $R_3$ représente un radical alkyle ($C_{1-4}$) thiométhyle, ainsi que de leurs sels d'addition avec les acides minéraux ou organiques pharmaceutiquement acceptables à l'obtention d'un médicament pour compenser le vieillissement ou la souffrance cérébrale.

2. Utilisation des dérivés de formule (I) selon la revendication 1, dans laquelle R représente un atome d'hydrogène, ainsi que de leurs sels d'addition avec les acides minéraux ou organiques pharmaceutiquement acceptables, à l'obtention d'un médicament pour compenser le vieillissement ou la souffrance cérébrale.

3. Utilisation des dérivés de formule (I) selon la revendication 1 ou 2, dans lesquels $R_1$ représente un atome d'hydrogène, ainsi que de leurs sels d'addition avec les acides minéraux ou organiques pharmaceutiquement acceptables, à l'obtention d'un médicament pour compenser le vieillissement ou la souffrance cérébrale.

4. Utilisation de la 6-méthyl 8-(méthylthiométhyl) ergoline ainsi que ses sels d'addition avec les acides minéraux ou organiques pharmaceutiquement acceptables, à l'obtention d'un médicament pour compenser le vieillissement ou la souffrance cérébrale.

5. Compositions pharmaceutiques pour compenser le vieillissement ou la souffrance cérébrale renfermant à titre de principe actif l'un au moins des dérivés tels que définis à l'une des revendications 1 à 4 ou l'un au moins de ses sels d'addition avec les acides minéraux ou organiques pharmaceutiquement acceptables.

**Claims**

1. The use of ergoline derivatives of formula (I):

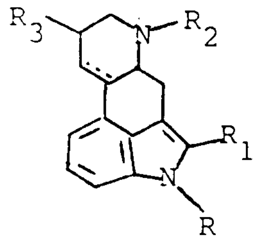

(I)

in which R represents a hydrogen atom or an alkyl radical containing 1 to 4 carbon atoms, $R_1$ represents a hydrogen, chlorine or bromine atom, $R_2$ represents a hydrogen atom or an alkyl radical containing 1 to 4 carbon atoms, $R_3$ represents a $(C_{1-4})$ alkyl thiomethyl radical, as well as their addition salts with pharmaceutically acceptable mineral or organic acids to obtain a medicament to compensate for ageing or cerebral pain.

**2.** Use of derivatives of formula (I) according to claim 1, in which R represents a hydrogen atom, as well as their addition salts with pharmaceutically acceptable mineral or organic acids to obtain a medicament to compensate for ageing or cerebral pain.

**3.** Use of derivatives of formula (I) according to claim 1 or 2, in which $R_1$ represents a hydrogen atom, as well as their addition salts with pharmaceutically acceptable mineral or organic acids to obtain a medicament to compensate for ageing or cerebral pain.

**4.** Use of 6-methyl 8-(methylthiomethyl) ergoline as well as its addition salts with pharmaceutically acceptable mineral or organic acids to obtain a medicament to compensate for ageing or cerebral pain.

**5.** Pharmaceutical compositions to compensate for ageing or cerebral pain containing as active ingredient at least one of the derivatives defined in one of claims 1 to 4 or at least one of its addition salts with pharmaceutically acceptable mineral or organic acids.

**Patentansprüche**

**1.** Verwendung der Ergolinderivate der Formel (I)

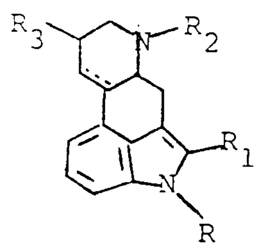

(I)

worin R für ein Wasserstoffatom oder einen Alkylrest mit 1 bis 4 Kohlenstoffatomen steht, $R_1$ ein Wasserstoff-, Chlor- oder Bromatom bedeutet, $R_2$ ein Wasserstoffatom oder einen Alkylrest mit 1 bis 4 Kohlenstoffatomen wiedergibt, $R_3$ für einen Alkyl$(C_{1-4})$-thiomethylrest steht, sowie von deren Additionssalzen mit pharmazeutisch verträglichen Mineral- oder organischen Säuren zur Erzielung eines Arzneimittels für die Kompensation der Alterung oder des cerebralen Leidens.

**2.** Verwendung der Derivate der Formel (I) gemäß Anspruch 1, worin R für ein Wasserstoffatom steht, sowie von deren Additionssalzen mit pharmazeutisch verträglichen Mineral- oder organischen Säuren zur Erzielung eines Arzneimittels für die Kompensation der Alterung oder des cerebralen Leidens.

**3.** Verwendung der Derivate der Formel (I) gemäß Anspruch 1 oder 2, worin $R_1$ für ein Wasserstoffatom steht, sowie von deren Additionssalzen mit pharmazeutisch verträglichen Mineral- oder organischen Säuren zur Erzielung eines Arzneimittels für die Kompensation der Alterung oder des cerebralen

Leidens.

**4.** Verwendung von 6-Methyl-8-(methylthiomethyl)-ergolin sowie von dessen Additionssalzen mit pharmazeutisch verträglichen Mineral- oder organischen Säuren zur Erzielung eines Arzneimittels für die Kompensation der Alterung oder des cerebralen Leidens.

**5.** Pharmazeutische Zusammensetzungen zur Kompensation der Alterung oder des cerebralen Leidens, enthaltend als Wirkstoff zumindest eines der Derivate gemäß einem der Ansprüche 1 bis 4 oder zumindest eines ihrer Additionssalze mit pharmazeutisch verträglichen Mineral- oder organischen Säuren.